Europäisches Patentamt

European Patent Office

Office européen des brevets

(11) Publication number: **0 293 827**
**A2**

# EUROPEAN PATENT APPLICATION

(21) Application number: 88108686.2

(22) Date of filing: 31.05.88

(51) Int. Cl.⁴: **G01N 33/569 , A61K 39/008**

(30) Priority: 01.06.87 IL 82740

(43) Date of publication of application:
07.12.88 Bulletin 88/49

(84) Designated Contracting States:
AT BE CH DE ES FR GB GR IT LI LU NL SE

(71) Applicant: YEDA RESEARCH AND
DEVELOPMENT COMPANY, LTD.
Kiryat Weizman P.O. Box 95
Rehovot 76100(IL)

(72) Inventor: Jaffe, Charles
24 Marginit Street
Yavne 70600(IL)
Inventor: Zalis, Mariano
Ge. San Martin 1290 Apt.1004 Leblon
Rio de Janeiro(BR)

(74) Representative: Vossius & Partner
Siebertstrasse 4 P.O. Box 86 07 67
D-8000 München 86(DE)

(54) Assay for leishmaniasis.

(57) A purified protein derived from Leishmania donovani, for use as antigen in an assay for the determination of Leishmaniasis and for use in vaccines against this disease. Such proteins react with monoclonal antibodies raised against Leishmania donovani. Identical or equivalent proteins can be produced by recombinant DNA technology. Instead of the proteins there can also be used effective segments thereof.

EP 0 293 827 A2

## "ASSAY FOR LEISHMANIASIS"

### BACKGROUND OF THE INVENTION:

The diagnosis of visceral leishmaniasis (VL) is routinely made by examining bone marrow aspirates or spleenic needle biopsies for the presence of amastigotes. These procedures can be dangerous. These invasive procedures are ofter unsuccessful and frequent biopsies maybe necessary to identify the parasites in stained tissue smears or by culturing of the parasites. This disease, fatal if not rapidly diagnosed and treated, is characterized by very high titers of both nonspecific and specific antibodies. Taking advantage of the elevated antibody levels serologic tests, such as the indirect immunofluorescence (IFA), enzyme-linked immunosorbent (ELISA) or radioimmunoassay (RIA) have been developed. These methods are sensitive and are slowly acquiring importance as complementary procedures to existing invasive techniques for the diagnosis of VL. While serodiagnostic assays may be reliably used for the rapid screening of populations under optimum conditions, in areas free from Chagas' disease, significant problems have restricted their widespread use in areas coendemic for both diseases. Positive crossreactions between the crude parasite antigen and sera, primarily from Chagas' disease, sleeping sickness, mucocutaneous leishmaniasis and to a lesser extent lepromatous leprosy, malaria or cutaneous leishmaniasis complicate diagnosis.

### SUMMARY OF THE INVENTION:

The invention relates to a novel serodiagnostic assay for VL based on purified Leishmania donovani proteins. This assay is simple, easily standardized and completed with 3 h. More than 95% of the sera examined were correctly identified as positive or negative for VL.

Sera were obtained from normal endemic or non-endemic controls and from patients clinically diagnosed for Chagas' disease, cutaneous, mucocutaneous or visceral leishmaniasis, lepromatous leprosy, malaria, schistosomiasis, African trypansosmiasis, ameobiasis and systemic lupus erythematosus. Sources of the sera are summarized in Table 1.

### Summary of Preparation of Parasitic Antigen:

Two different isolates of Leishmania donovani (Edmael and LV9, obtained courtesy of Dr. Steve Reed, Cornell University Medical School, N.Y. and Dr. Marcel Hommel, Liverpool School of Tropical Medicine and Hygiene, England, respectively) were used for purification of the parasite proteins. Promastigotes were grown in Schneider's Drosophilia medium containing 15% calf serum (Seromed Lot # OHOI). Membranes were prepared by nitrogen cavitation and differential centrifugatation. Membranes were solubilized in 20 mM Tris-HCl (pH 8.0) containing 0.15 M NaCl, 0.5% sodium deoxycholate, 10 mM EDTA, 2 mM iodoacetamide, 1 mM phenylmethylsulfonylchloride and 1 mM leupeptin for 1/2 h at 0°C. Nonsolubilized material was removed by centrifugation for 1/2 h at 48,000xg. The two proteins gp70-2 and dp72 were purified by affinity chromatography on monoclonal antibodies LXXVIII 235-A8 (D2) and LI 3H2-G6 (D13) (Jaffe et al., J. Immunol. 133, 440-447 (1984)) respectively, coupled to ε-aminocaproic acid derivatized Sepharose 4B and activated. The purity of each antigen preparation was monitored by silver staining following sodium dodecylsulfate polyacrylamide gel electrophoresis.

In the following there is described the purification and preliminary characterization of the two membrane proteins recognized by antibodies D2 and D13, gp70-2 and dp72 respectively, from L. donovani. The purified proteins are highly antigenic and are recognized by both dog and human VL sera. They are useful in the rapid serodiagnosis of visceral leishmaniasis.

### Materials and Methods:

## Leishmania.

Leishmania donovani promastigotes were subcultured biweekly in Schneider's Drosophila medium containing 20% fetal calf serum (Seromed Lot # OHOI). Labeling of the promastigotes with [35S]-methionine (Amersham, 1125 Ci/mmole) was carried out by preincubating $5 \times 10^7$ log phase promastigotes for 1 h in Schneider's medium without methionine, yeast extract and fetal calf serum. Methionine (250 μCi) was added and 1 h later the medium was adjusted to 20% with dialysed fetal calf serum. The cells were further incubated 10-12 h at 26°C and extensively washed with sterile 50 mM phosphate buffered saline, pH 7.2 (PBS).

## Membrane and cell lysates.

Crude membrane fractions were prepared by nitrogen cavitation and differential centrifugation. In brief, harvested parasites ($1-5 \times 10^{10}$) were washed once in PBS (15 min x 2000 rpm) and resuspended at 4°C in lysis buffer (20 mM Tris-HCl, 40 mM NaCl, pH 7.4) containing the following proteolytic inhibitors: 10 mM sodium ethylenediaminetetracetate, 2 mM iodoacetamide, 2 mM phenylmethylsulfonyl fluoride and 1 mM leupeptin. The cells were disrupted in a Parr cell disruption bomb (10 minutes incubation at 1500 psi). Cell lysates were then separated into three fractions by centrifugation (Sorval SS-34 rotor): fraction I (pellet 4,350xg, 10 minutes); fraction II (pellet 39,000xg, 30 minutes) and fraction III (supernatant 39,000xg, 30 minutes). Fraction II was primarily used throughout this study.

Whole cell lysates were prepared by washing promastigotes ($2-10 \times 10^8$) cells) twice with PBS and resuspending them in 1-2 ml of lysis buffer containing the same proteolytic inhibitors as described above. The cell suspension was sonicated briefly (10-15 seconds) at 4°C in a water bath sonicator and snap frozen in liquid $N_2$. Lysates were stored at -70°C until use.

## Affinity resin.

Monoclonal antibodies D2 and D13 were purified from BALB/c mouse ascites fluid by hydroxylapatite chromatography and dialyzed against 0.2 M sodium bicarbonate buffer (pH 8.5). Coupling of the antibody to a p-nitrophenyl derivative of either ε-caprioc/β-alanine Sepharose 4B (Pharmacia) or Trisacryl GF2000 (Pierce) was carried out 48-72 h at 4°C according to Wilchek and Miron (Wilchek et al., Limitations of N-hydroxysuccinimide esters in affinity chromatography and protein immobilization; Biochemistry, in press (1987)). The amount of protein coupled was 5 mg/g for Sepharose 4B and 3 mg/g for Trisacryl GF2000.

## Purification of the membrane proteins.

Membranes (2 mg) from either L. donovani infantum (LV9; MHOM/ET/67/L82 or L.d. chagasi (Edmael; MHOM/BR/00/BA2) were dissolved in 30 mM Tris-HCl, pH 8.2 containing 0.5% (w/v) sodium deoxycholate (DOC) and the same proteolytic inhibitors described above for membrane preparation. After 1 h at 4°C the material was centrifuged at 39,000xg for 30 minutes and the supernatant removed. The supernatant (8 ml final volume) was incubated batchwise with the affinity resin in a 50 ml tube for 2 h at 4°C with constant shaking.

After binding, the resin was washed by centrifugation (5 minutes, 1500 x g) twice with 0.05% Tween-20 in 30 mM Tris•HCl, pH 8.2; twice with 0.5% DOC in the same Tris buffer and loaded into a mini-column (Isolab, Inc) for elution. The bound protein was eluted with 50 mM diethylamine, pH 11.5 containing 0.01% DOC into a microfuge tube containing 100 μl of 2 M Tris-base or solid glycine. The column was re-equilibrated by washing with 30 mM Tris-HCl, pH 8.2, 0.1% DOC.

Several preparations of protein were further purified by size exclusion high performance liquid chromatography (SE-HPLC). Material from the affinity column was separated on a 7.5 x 3000 mm TSK G3000 SW column 10 μm particle size (LKB). The column was equilibrated with 0.1 M PBS containing 0.1% (w/v) sodium dodecyl sulfate (SDS). Fractions (2 ml) were collected at a rate of 1 ml $min^{-1}$. Eluted protein was stored at either 4°C or -70°C until use. In some cases material was dialyzed extensively against double distilled water at 4°C.

3

SDS-polyacrylamide gel electrophoresis (PAGE) and western blotting.

SDS-PAGE was carried out in 5-12% gradient gels or 10% and 15% nongradient gels. Polyacrylamide gels for silver staining were washed extensively in 50% methanol-water before staining. Gels containing [35S]-methionine labelled material were treated with Amplify (Amersham), dried and exposed to X-ray film (Agfa Curix) at -70° C.

Proteins from polyacrylamide gels for western blotting were transferred electrophoretically (2 hrs, 40V) onto 0.45 μm nitrocellulose paper (Schleicher & Schuell) immediately without fixing. The nitrocellulose was quenched with 0.3% Tween-20 in PBS and washed twice with washing buffer (0.05% Tween-20 in PBS). The blots were incubated 2 hours at room temperature with the monoclonal antibodies (1/4 dilution in washing buffer) and excess antibody removed by rinsing 4 times with washing buffer (15 minutes/each). The paper was probed for 1 hour with [125I]-rabbit F(ab')2 anti-mouse IgG (Zymed) and rinsed 4 times with washing buffer. The strips were air dried and exposed to X-Ray film at -70° C using an intensifying screen (Dupont).

Peptide mapping.

Partial proteolyic maps of the two proteins were carried out by Cleveland digests. Cleveland D.W. in Mehtods in Enzymology (Fleischer, S. and Fleischer, B., ed.), vol 96, 222-229, Academic Press, New York, (1983). Proteins from promastigotes labelled with [35S]-methionine were purified by affinity chromatography and separated by SDS-PAGE on 10% polyacrylamide gels. The labeled material was excised from the gel and coelectrophorised on a second 15% polyacrylamide gel with sample buffer containing Staphylococcus aureaus, strain V8 protease (Sigma). After stacking the current was turned off for 40 minutes and the run continued until the tracking dye reached the bottom of the gel.

Enzyme-linked immunosorbent assay (ELISA)

An ELISA to determine the best source of antigen was carried out. The concentration of parasite lysates absorbed overnight to polyvinylchloride microtiter plates was 80,40 or 20 μg/ml. The plates were blocked with 3% fetal calf serum in PBS/0.02% NaN3 for 10 hours at 4° C. Undiluted hybridoma culture supernatants (50 μl) were incubated with the antigen. This concentration of antibody was shown to be saturating at the highest protein concentration used. After 12 hours at 4° C excess unbound antibody was removed by washing 4 times with 0.1% Tween-20/PBS. The plates were incubated for 1 hour at 37° C with sheep F-(ab')2 antimouse-horseradish peroxidase conjugate (Amersham), washed 4 times with 0.1% Tween 20/PBS and the color developed by incubating with the substrate 2,2' azino-di-(3-ethyl-benzthiazoline sulfonate) for 1 hour.

The periodate - ELISA was carried out by modifying the proceeding assay prior to incuabtion with the antibody culture supernatants. Antigen coated plates (50 μg/ml) were blocked overnight and washed 3 times with 0.1% Tween-20/PBS. Following one quick rinse with 50 mM sodium acetate buffer (pH 4.5), 0.5 mM to 10 mM sodium periodate (50 μl) in acetate buffer or just acetate buffer alone were added to each well. After 1 hour at 25° C the reaction was terminated by washing with acetate buffer and adding a 1% glycine solution in PBS (100 μl) for 30 minutes. The plates were washed 3 times with PBS and the assay continued by incubating with the monoclonal antibody.

A dot-ELISA was carried out by spotting 2 μl of the purified proteins onto 0.2 μm nitrocellulose paper. The matrix was quenched with 0.3% Tween-20 in PBS for 1 hour at room temperature and washed twice in washing buffer. The spotted antigens were incubated in human sera (1/10 dilution in washing buffer) for 1 hour at room temperature followed by 4 rinses (20 minutes each) in washing buffer. After probing for 1 hour with the peroxidase-F(ab')2 conjugate the paper was rinsed 4 times with washing buffer and the reactions developed 15 minutes with the substrate 4-chloro-1-naphtol. The substrate was prepared fresh (3 mg/ml in anhydrous methanol diluted 1 to 6 in Tris-buffered saline) and 30% H2O2 (1 μl/ml) added just prior to use.

Amino acid analysis.

Amino acid analyses of the purified proteins were carried out on a Durham D-500 by the Biological Services Department of the Weizmann Institute after hydrolysis in vacuo for 16 hours with 6N HCl.

Selection of antigen source.

The L. donovani isolate for antigen purification was selected by measuring antibody binding to whole promastigote lysates. Monoclonal antibody concentration was maintained constant and the total parasite protein for each lysate varied. The isolate giving maximum antibody binding at minimum protein concentration was chosen as starting material for the purification of each respective protein.

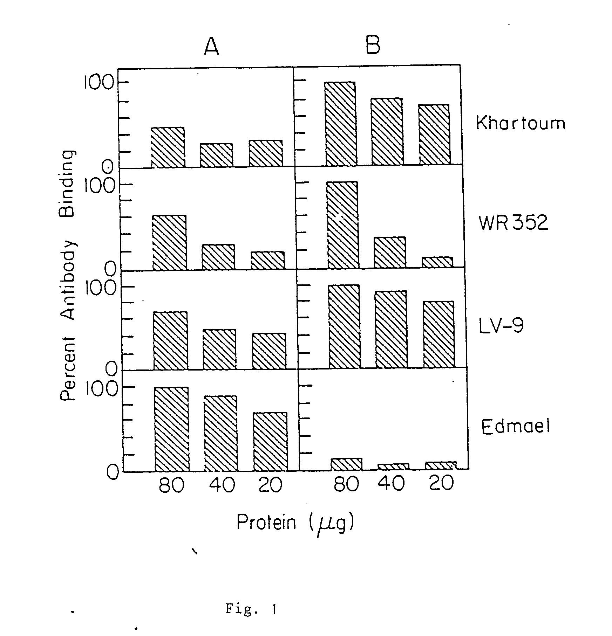

Four L. donovani stocks were examined prior to purification: Two L.d. donovani, one from India (WR352) and one from Africa (Khartoum); one L.d.infantum (LV9-Africa) and one L.d. chagasi (Edmael-Brazil). Antibody D2 was produced against membranes of Edmael (ibid.), Of the four stocks examined this antibody reacts best with whole lysates of Edmael (Figure 1), approximately 1.7 fold higher at 20 μg/ml protein than LV9. Binding to Khartoum and WR352 was poor. The difference between Emael and the other stocks diminished at higher protein concentration (80 μg/ml) but remained significant. Likewise a significant variation in the binding of D13 to different L. donovani stocks was observed (Figure 1B). This monoclonal antibody was produced against membranes of a L. donovani stock (WR168c) isolated in Africa(ibid.) Binding to LV9 and Khartoum, two African isolates, was approximately equal at all protein concentrations examined and significantly higher than for Edmael or WR352 at 20 μg/ml. At higher protein concentrations (80 μg/ml) only Edmael demonstrated poor binding, approximately 5 fold less than the other stocks tested. Based on these results the protein reacting with D2 was purified from membranes of Edmael and the protein reacting with D13 from membranes of LV9.

Selection of antigen soma.

Intracellular amastigotes purified from hamsters infected with the Khartoum isolate of L. donovani were also screened for reactivity with both rabbit polyclonal antibodies to the purified proteins (gpt70-2 and dp72) and the monoclonal antibodies. By several techniques, immunofluorescence, western blotting, ELISA, and immunoprecipitation of $^{35}$S-methionine labeled parasites, both proteins gp70-2 and dp72 were shown to be present in amastigotes.

Purification of the proteins recognized by D2 (gp70-2) and D13 (dp72).

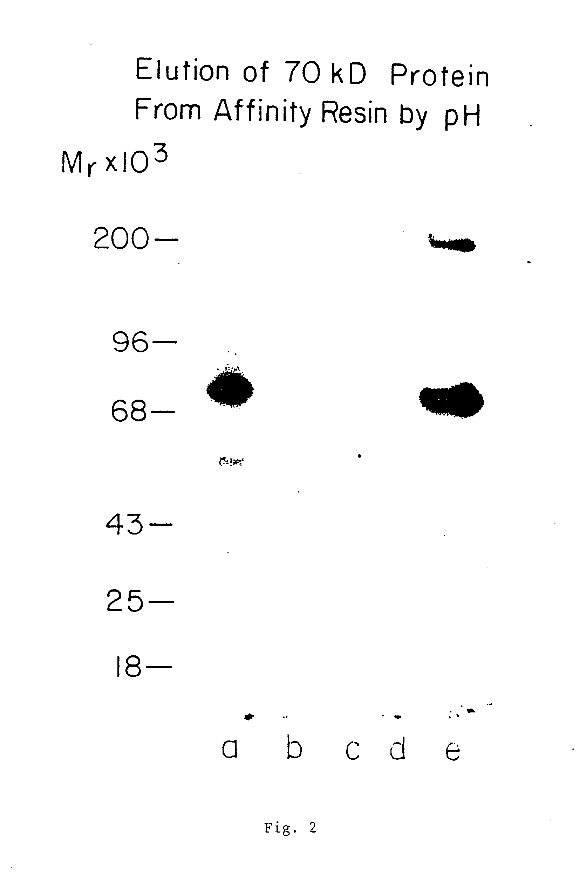

Since neither protein was labelled by the Na($^{125}$I)-lactoperoxidase procedure on intact promastigotes (Jaffe et al. (1984) ibid), purification of the proteins was followed by either dot-ELISA or western blotting following SDS-PAGE of the eluted material. Conditions for eluting the bound protein from the monoclonal antibody affinity resins were examined (Figure 2). Several different conditions were tested: 1 M acetic acid, pH 2.2, 0.01% Triton X-100; 0.1 M glycine, pH 2.5., 0.01% Triton X-100; 50 mM diethylamine, pH 9.5 or pH 11.5 containing 0.01% DOC. Only the high pH 11.5 buffer (Fig. 2e) eluted antigenic material from the D13-affinity resin. Identical elution characteristics were observed with the D2 affinity resin (data not shown). This material migrated at Mr 72,000 in SDS-PAGE identical to the original antigen which reacts with D13 in the membranes used for purification (Figure 2 lanes a and e). No other antigenically active material was identified in the eluted fraction (lane e), except for a high molecular weight band which may represent mouse monoclonal antibody leakage from the resin. The band reacting with D13 (Mr 50,000) in the solubilized membranes prior to affinity chromatography (lane a) may represent proteolysis of the 72,000 protein.

Silver staining after SDS-PAGE of material eluted from the Sepharose 4B affinity resins and the original solubilized membrane fraction showed a considerable purification of the antigens recognized by D2 (Insert - Figure 3, lanes A and B) and D13 (data not shown). However some nonspecific binding to the affinity resin was also observed. Further purification of the D2 binding protein gp70-2 was obtained by SE-HPLC. As shown in Figure 3, only the material eluting at 11-12 minutes post injection was antigenically active. This fraction reacted with D2 monoclonal antibody in a dot-blot was devoid of other bands by SDS-PAGE silver staining (Figure 3, lane C). The Mr 49,000 band in all the lanes is a contaminant present in the gel sample buffer (lane D).

Further reduction in nonspecific binding to the resin was accomplished by addition of a high salt wash prior to elution from the column and the substitution of Sepharose 4B by Trisacryl GF2000 as the supporting matrix. Silver staining after SDS-PAGE showed that both proteins migrate with similar molecular weights and are relatively free of contaminating material (data not shown).

Comparison of purified proteins.

The proteins purified by both affinity resins have very similar Mr on SDS-PAGE as examined by silver staining and western blotting. The relationship between the two proteins was unclear. The proteins may be identical, different or cross contaminated with each other, therefore their properties were compared by several techniques. Western blotting was carried out using each purified protein and several monoclonal antibodies. The starting membrane fraction was also blotted in parallel to the purified proteins (Figure 4A and B). Antibodies D2, D13 and X4 all reacted with the starting membrane fraction (Figure 4A and B, lanes c, e and g). Monoclonal antibody X4 shows binding to multiple leishmanial components (Jaffe and Safstein, submitted), but does not react with gp70-2 or dp72. Lanes g and h are overexposed, so as to emphasize the absence of binding by X4 to the purified proteins. When dp72, the material eluted from the D13 affinity resin was probed, only antibody D13 reacts (Figure 4A, lane d). No binding was seen with either D2 or X4 to this material (Figure 4A, lanes f and h). Similarly when gp70-2, the material eluted from the D2 affinity column, was incubated with D2, D13 and X4, only D2 reacts (Figure 4B, lanes d, f and h). These results demonstrate that only D2 recognizes material isolated on the D2 affinity resin and that only D13 reacts with material purified on the D13 resin. No crossreactivity was seen between the protein purified on one antibody resin and the heterologous antibody.

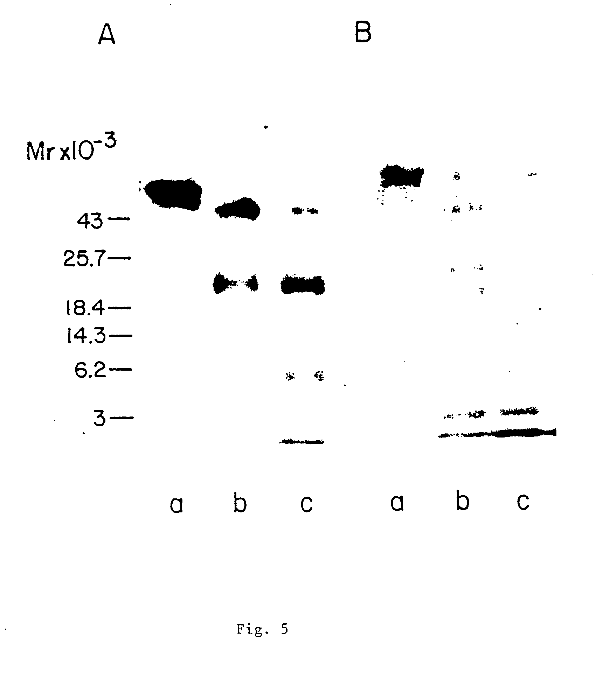

A Cleveland digest was carried out using ($^{35}$S)-methionine labelled material purified by affinity chromatography on the respective resins and SV8 protease (Figure 5). Both proteins were sensitive to digestion with the enzyme, however the overall pattern obtained was different. Digestion of Dp72 with SV8 protease produces two major bands of Mr approximately 45,000 and 20,000, and additional fragments between 4-6000. gp70-2 upon digestion with this enzyme shows multiple polypeptides of approximately equal intensity. The fragments obtained differ in Mr from those produced with dp72. Preliminary amino acid analysis showed no striking differences between the two proteins (Table I).

Using rabbit polyclonal antibodies against gp70-2 and dp72 and the monoclonal antibodies D2 and D13, additional isolates of L. donovani and other species of Leishmania were examined by western blotting. The protein gp70-2 demonstrated little size heterogeneity among different isolates of L. donovani. This protein was not identified in L. major, L. tropica, L. mexicana, or L. aethiopica. Interestingly dp72 showed significant variation in molecular weights between new and ld world isolates of L. donovani. This protein in three isolates of L. donovani chagasi from Brazil migrated at 66,000 daltons. In addition, antibodies to dp72 also recognized a homologous protein in isolates in cutaneous forms of the disease even though little antibody reaction against these antigens are observed by dot-blot ELISA (see next section).

Effect of periodate oxidation.

Some preliminary characterization of the epitope(s) recognized by the monoclonal antibodies was carried out. Binding of the monoclonal antibodies D2 and D13 to periodate treated membranes was examined over a range of concentrations (Figure 6). Sodium periodate under the conditions utilized oxidates the vinicinal hydroxyl groups of carbohydrates. Only the binding of antibody D2 to membranes was sensitive to this treatment. At 5 mM and 10 mM periodate, respectively, 37.5% and 75% of the antibody binding was inhibited. No effect was observed on the binding of D13 or X4 at all concentrations examined. This finding suggests that the epitope recognized by antibody D2 contains carbohydrates.

Reactivity of proteins with human sera.

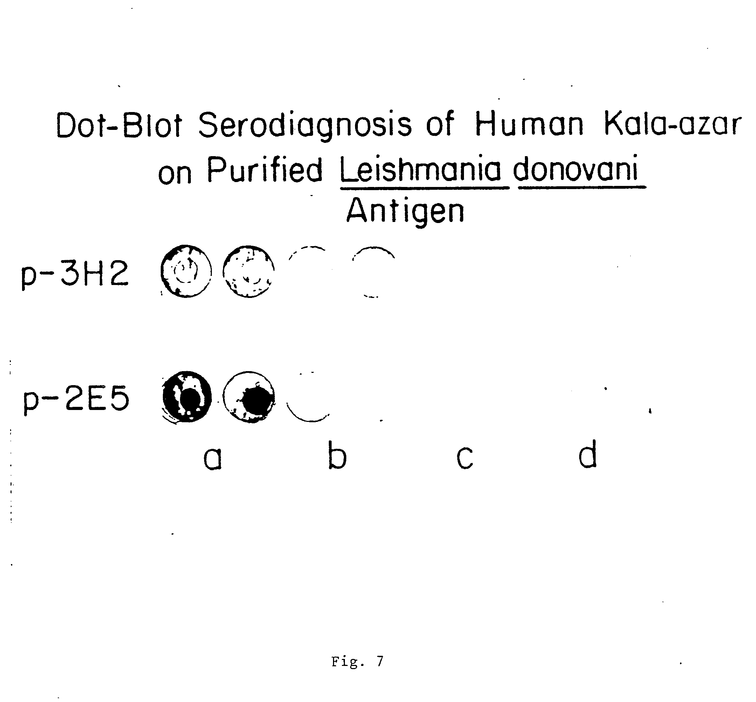

The binding of human sera to the pure proteins was examined by a dot-ELISA. The pure proteins (300 ng/dot dp72 and 150 ng/dot gp70-2) were dotted on nitrocellulose. Sera from a normal endemic control and patients with visceral leishmaniasis, Chagas' disease and cutaneous leishmaniasis were incubated with the pure protein at a 1/10 dilution. Only the serum from the patient with visceral leishmaniasis reacted with both pure proteins (Figure 7). No crossreaction was observed with the other sera tested.

Reactivity of proteins with human T cells.

Antigen specific T cell proliferation of peripheral blood leukocytes obtained from cutaneous leishmaniasis was initiated using dp72 and gp70-2. The two patients examined demonstrated stimulation indexes with dp72 of 3 and 20 times the control subject. No T cell proliferation in these patients was observed to gp70-2. These results suggest that dp72 induces a T cell response of the species L. tropica, L. mexicana and L. major, suggesting that dp72 or a similar protein is also present in other species of Leishmania.

## Dot Blot Serodiagnostic Assay.

The purified antigen ($2\mu$l in 0.5% sodium deoxycholate, 20 mM Tris-HCl pH 8.0) was spotted onto nitrocellulose paper (Schleichler and Schuell, GmbH. W. Germany, 0.45 $\mu$m) and allowed to air dry for 5-10 minutes. The volume of antigen dotted for each test represents 150ng gp70-2 and 300ng dp70. Protein concentration was estimated using the BioRad protein assay reagent (BioRad Laboratories, Richmond, Ca). The paper was quenched for 1 hour at room temperature using a blocking agent (0.3% Tween 20 in 50 mM phosphate saline buffer, pH 7.2 - PBS) and washed 2X with 0.05% Tween 20 in PBS (PBS -Tween). The nitrocellulose paper was aligned and clamped into a dot blot apparatus, either Bio-dot microfiltration apparatus (BioRad Laboratories Richmond, Ca.) or a homemade apparatus (Zalis and Jaffe, in preparation) prepared by drilling 1.7 mm diameter holes in a 96 well flat bottom polystyrene microtiter plate. Incubation with human sera ($50\mu$l in duplicate of a 1/100 dilution in PBS-Tween) were carried out for 1 hour at RT. The paper was then thoroughly rinsed with PBS-Tween (4X) and 50 $\mu$l of a solution containing protein A - horseradish peroxidase conjugate (Amersham International, England) diluted 1/2000, in 1% bovine serum albumin, PBS-Tween was added. After 30 minutes the nitrocellulose was rinsed quickly with PBS-Tween, washed 3X (20 minutes each) and the papers incubated in a substrate solution. This solution contained 4-chloro-1-napthol (3 mg/ml) in anhydrous methanol) diluted 1/6 in 50 mM Tris, 500 mM NaCl, pH 7.4. Hydrogen peroxide (30% $H_2O_2$; $1\mu$l/ml substrate) was added just prior to use. The reaction was terminated by rinsing the papers in water and allowing to air dry before analyzing the results. All experiments were carried out as double blind assays.

Titration of Human Sera.

Titrations of human sera binding to L. donovani membranes were performed by ELISA. The antigen coated (0.08 $\mu$g/ml protein) polyvinyl chloride plates were blocked overnight with 0.3% Tween 20 in PBS and rinsed with PBS-Tween (3X). After incubation for 1 hour with selected sera diluted in PBS-Tween the plates were washed (3x PBS-Tween) and incuabted with $50\mu$l of protein A-horseradish perozidase conjugate. Excess conjugate was removed after 30 minutes at $37°$C and the plates were washed extensively with PBS-Tween. A solution containing (100 $\mu$l) was added to each well and the absorbance ($\lambda_{405nm} - \lambda_{490nm}$) measured using a Bio-tek 310 Microplate Reader (Burlington, VT).

Assays showed them to be antigenically active and bind serum from a patient with visceral leishmaniasis. The optimum concentration of antigen (gp70-2 and dp72) and serum dilution were determined. The reaction of two reference sera, one from a patient with VL and a second from a patient with Chagas' disease were titered using different concentrations of antigen (Figure 1). No reaction was visible with the Chagas' disease serum even at the highest antigen concentrations (150 mg/ml) or serum dilution (1/10) examined. The VL serum from India which shows a titer on crude membranes of ~$10^{-5}$ reacted strongly with both antigens. A positive reaction was visible using a 1/50 serum dilution out to 37.5ng of either antigen. Protein gp70-2 reacted more strongly than dp72 and could be detected even at a 1/500 serum dilution using 75ng of antigen.

Similar titrations using the VL reference serum were carried out to standardize every newly purified batch of antigen. All lots examined to date showed identical characteristics. Working conditions of 300ng dp72; 1/50 serum dilution and 150ng gp 70-2; 1/100 serum dilution were chosen for all the following assays.

Composite figures prepared from part of the results obtained in assays carried out with both VL and other sera are given in figures 2. A total of 128 sera were examined by dot blot for reactivity with antigen gp70-2 and 92 sera for binding to antigen dp72. All assays were carried out in duplicate and evaluated without any foreknowledge of the sera type. Each 8X12 antigen dot matrix on nitrocellulose tested always included two negative controls, normal human and Chagas' sera, as well as a reference visceral leishmaniasis serum. All dots were evaluated with respect to these internal controls. Positive reactions appear as compact purple dots on a white background. The intensity of the reaction varies considerably between different VL serum, but does not fluctuate upon repeated tested of a single sample on either the same dot matrix (note duplicates) or different antigen matrixes. Positive reactions can be weak (Figure 2a B4, C1, D2; Figure 2b C1, E1, G2) or very strong (Figure 2a B3, E3; Figure 2b A6). Negative reactions appear white and show no dot of precipitate, though occasionally a halo may be present (Figure 2a E6; Figure 2b G5). This halo represents a nonspecific precipitation, perhaps due to incomplete removal of the enzyme before addition of substrate and was primarily found in assays carried out using the home-made apparatus.

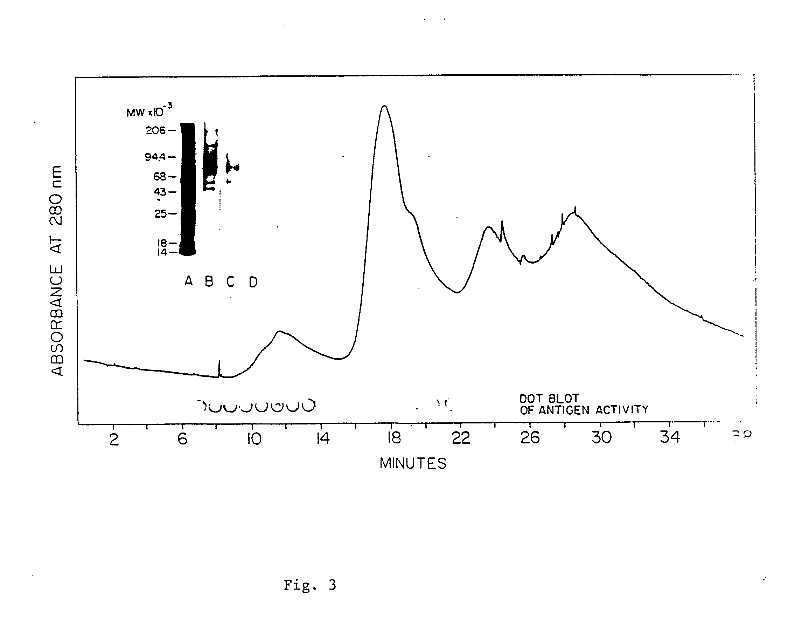

Several sera from suspected but unconfirmed cases of visceral leishmaniasis originating in Africa, Columbia and Brazil were negative when tested for binding to dp72 and gp70-2. Since high anti-leishmanial antibodies are characteristic of visceral leishmaniasis, we decided to measure the binding of these sera to crude parasite membrane antigen from *L. donovani* promastigotes (Figure 3). Only one of the 5 sera which failed to react with the purified parasite proteins gp70-2 and dp72 (Mandel-Brazil) showed a titer($\sim 10^{-5}$) comparable to the reference VL sera. The remaining sera which tested as negative all gave titration curves identical to the negative control normal human sera (Figure 3). These results suggest that the suspected VL sera missed by the dot-ELISA on dp72 and gp70-2 were either from patients without VL or mislabelled.

A summary of the results obtained with each antigen dot-ELISA is given in Tables II and III. Using antigen gp70-2 36/40 VL sera (90%) were correctly diagnosed; 10% were misdiagnosed. Only 1/86 (1.2%) of the non-VL sera (a new world cutaneous leishmaniasis serum) was read as a false positive. Assays carried out using the second antigen, dp72, diagnosed correctly 21/21 VL sera (100%). However the number of false positives with this antigen were slightly higher than for gp70-2, perhaps because the reactions were weaker with VL sera. A total of 5/71 sera (7.0%) were misdiagnosed as false positives. The cases included 3 cutaneous leishmaniasis, 1 Chagas' disease and 1 lepromatous leprosy case. Only one cutaneous leishmaniasis serum (from Columbia) which gave a false positive with dp72 was also misdiagnosed on gp70-2. All of the other sera misdiagnosed by dp72 were correctly diagnosed by gp70-2. Interestingly, 2 VL sera misdiagnosed by gp70-2 as false negatives were correctly diagnosed by dp72. Together greater than 95% of the sera were correctly diagnosed as positive or negative for visceral leishmaniasis.

Methods based on serum antibody reactions with whole parasite antigen, such as ELISA and IFA, though slowly becoming accepted are still not routine procedures. In Kenya a direct ELISA on parasite antigen has been reliably used for the diagnosis of VL.[7,8,10] False positive reactions were found with other sera at low serum dilutions, but by diluting patient sera 1/800 or 1/1000 good results were obtained in two separate studies with 63 and 7 VL patients, respectively.[8,10] No crossreactions were observed at these dilutions with other sera from malaria, schistosomiasis, leprosy, African trypanosomiasis and several other diseases. However, when tested with patient sera from Chagas' disease or mucocutaneous leishmaniasis strong crossreactions were observed.[10,11,18] Reactions in conventional serodiagnostic assays with these sera pose a problem of major significance in South America or in the diagnosis of patients returning from regions coendemic for VL, mucocutaneous leishmaniasis and Chagas' disease.

The dot-ELISA is equal to or more sensitive (90%, gp70-2; 100%, dp72) than existing assays. The specificity for VL by dot-ELISA is better than any existing assay (false positives - 1%, gp70-2; 7%, dp72). Only one crossreaction was noted with 15 sera from Chagas' disease at a 1/50 dilution on antigen dp72 and none with 19 sera at a 1/100 dilution on gp70-2. Even at a 1/10 serum dilution, when examined, no reaction was observed for Chagas' disease. The ability to use such highly concentrated sera in this assay without false positive reactions may allow the early serodiagnosis and treatment of VL in asymtomatic patients. Another dot-blot ELISA using formalin-fixed whole parasites exhibited crossreactions even at 1/512 sera dilutions for Chagas' disease and African trypanosomiasis.

Compared to other assays which use crude parasite as antigen this assay offers several unique advantages. This is the first assay for VL which uses pure parasite proteins. Each antigen preparation can be readily compared to previous ones. Standardization can be carried out by titration versus a reference sera; protein purity monitored biochemically and a titer vs protein concentration established for each batch ensuring reproducibility. The elimination of variability in antigen preparation and assays between laboratories by using well defined reagents and standard procedures will facilitate the interpretation of results and establishment of test parameters.

These pure proteins are antigenically stable and easily purified by affinity chromatography. One liter of parasites ($\sim 10^{10}$ cells) provides enough material for about 10,000 assays. Preliminary studies demonstrated that lypholization and storage at 4°C does not affect the antigens. An antigen dot-matrix on nitrocellulose was prepared and stored at 4°C for greater than 6 months with no change in reactivity (data not presented). Reactivity was measured by titrating two reference sera from VL and Chagas' disease. The stability of these antigens will simplify reagent distribution and promote widespread utilization of the test, since the reagent matrix can be stored until required.

Constant exposure to *Leishmania* in endemic regions results in normal serum reactions on parasites which are greater than those seen with sera from non-endemic regions. This requires that a regional negative baseline be established for normal controls in each area prior to large scale patient screening. In non-endemic areas this phenomena could complicate serodiagnosis of a patient returning from an extended residence in an endemic region. No background reactivity was observed with normal control serum from endemic regions using the gp70-2; dp72 dot-ELISA. Normal controls from both endemic and non-endemic regions gave identical negative baselines.

The dot ELISA compares well with the competitive ELISA. The latter measures the binding to parasite lysates of species specific monoclonal antibodies against dp72 and gp70-2. Inhibition of antibody binding by patient sera is considered positive. All of the sera tested in both assays gave identical results. However, the competition assay doesn't utilize purified parasite antigens and still requires at least 48 hours to complete, rather than the 3-4 hours required for the dot-ELISA. Both assays demonstrate the specificity of the humoral response in VL patients to the antigenic epitopes of these two proteins, whether presented as a crude or purified antigen. However, the role of these proteins in the disease process remained to be elucidated. Interestingly dp72 is present as a cryptic antigen in *L. tropica*, a species which generally causes cutaneous leishmaniasis, but has been isolated from rare cases of VL.

Patient follow up after drug treatment is often a problem with VL. The disappearance of symptoms or L.D. bodies from bone marrow and spleen can be indicative of cure. However, relapses can occur and repeated biopsies are required to confirm the disappearance of parasites which may have been initially difficult to identify. Standard serodiagnostic assays are also of little use in monitoring recovery, since serum antibody titers to crude antigen may remain elevated for up to a year. Yet, titers against specific purified proteins may drop quickly upon cure. Serum reactions against the two proteins gp70-2 and dp72 are excellent indicators of VL. If antibodies against these proteins disappear rapidly upon cure, the dot-ELISA will provide a sensitive method for monitoring patients following drug treatment. We hope to evaluate this possibility in future studies.

Use for vaccination.

The above defined proteins, or segments thereof which retain an adequate effectivity, can be used as an active ingredient in vaccines against Leishmaniasis. Such proteins can be obtained from Leishmania donovani, as set out above, or they can be produced by recombinant DNA technology.

The vaccines are administered in a conventional manner. The quantity of the protein per dosage will generally be in the range of about 0.1 mg to about 10 mg. When fractions of the protein are used, high dosages are required.

TABLE I

Amino acid composition of two L. donovani membrane proteins [*]

| Amino acid | Gp70-2 % | Dp72 % |
|---|---|---|
| Asp | 10.3 | 10.5 |
| Thr | 4.8 | 4.7 |
| Ser | 8.9 | 8.5 |
| Glu | 15.8 | 13.3 |
| Pro | 3.4 | 4.7 |
| Gly | 10.3 | 10.5 |
| Ala | 10.3 | 9.5 |
| Met | 5.7 | 4.1 |
| Ile | 2.7 | 2.9 |
| Leu | 7.9 | 6.7 |
| Tyr | 4.1 | 3.3 |
| Phe | 3.8 | 3.3 |
| His | 2.1 | 2.0 |
| Lys | 4.8 | 4.3 |
| Arg | 6.8 | 4.7 |

[*] Average of three analyses on 6N HCl hydrolysates

TABLE II

Results of Direct ELISA Using gp70-2

A. Visceral Leishmaniasis Sera

| ORIGIN | SERUM TESTED | # POSITIVE | % POSITIVE | #FALSE NEGATIVE | %FALSE NEGATIVE | SOURCE+ |
|---|---|---|---|---|---|---|
| Brazil | 22 | 19 | 86.4 | 3 | 13.6 | 1,2,4 |
| Colombia | 3 | 2 | 66 | 1 | 33 | 6 |
| India | 6 | 6 | 100 | 0 | 0 | 3 |
| Africa | 9 | 9 | 100 | 0 | 0 | 5,13 |
| Total | 40 | 36 | 90.0 | 4 | 10.0 | |

B. Sera from Normal Patients and Other Diseases

| SERA | SERUM TESTED | #FALSE POSITIVE | %FALSE POSITIVE | SOURCE+ |
|---|---|---|---|---|
| Cutaneous | | | | |
| (old world) | 5 | 0 | 0.0 | 14 |
| (new world) | 19 | 1 | 5.3 | 2,6 |
| Chagas' | 18 | 0 | 0.0 | 1 |
| Malaria | 6 | 0 | 0.0 | 3,8 |
| Leprosy | 4 | 0 | 0.0 | 7 |
| African trypanosomiasis | 5 | 0 | 0.0 | 12 |
| SLE* | 2 | 0 | 0.0 | 10 |
| Schistosomiasis | 3 | 0 | 0.0 | 9 |
| Endemic normal | 13 | 0 | 0.0 | 1,3 |
| Non-endemic normal | 3 | 0 | 0.0 | |
| Other | 8 | 0 | 0.0 | 3,5 |
| Total | 86 | 1 | 1.2 | |

* Systemic lupus erythematosus

+ Key:1 Drs. Rodolfo Teixeira and Roberto Badaro, Hospital Prof. Edgard Santos, Salvador Brazil; 2 Dr. Mauro Marzochi, Fundacao Oswaldo Cruz, Rio de Janeiro, Brazil; 3 Dr. C.P. Thakur, Patna Medical College, Bihar, India; 4 Dr. Richard Pearson, University of Virginia, Charlottesville, VA; 5 Dr. Frank Neva, LPD, NIH, Bethesda, MD; 6 Dr. Nancy Saravia, CIDEM-Tulane University, Cali, Columbia; 7 Dr. Patricia Rose, Hansen's Disease Control Program, Georgetown, Guyana; 8 Dr. Ian McGregor, England; 9 Dr. Allain Dessein, Centre D'Immunologie

INSERM-CNRS, Marseille, France; 10 Dr. Peter Schur, Peter Bent Brigham Hospital, Boston, MA; 11 Dr. David Mirelman, Weizmann Institute of Science, Rehovot, Israel; 12 Dr. de Raadt, Trypanosomiasis Unit, W.H.O., Geneva, Switzerland; 13 Dr. M. Miles, London School of Hygiene and Tropical Medicine, London, England; 14 Dr. Justin Passwell, Chaim Sheba Medical Center, Tel Aviv, Israel.

TABLE III

Results of Direct ELISA Using dp72

A. Visceral Leishmaniasis Sera

| ORIGIN | SERUM TESTED | # POSITIVE | % POSITIVE | #FALSE NEGATIVE | %FALSE NEGATIVE | SOURCE[+] |
|--------|--------------|------------|------------|-----------------|-----------------|-----------|
| Brazil | 13 | 13 | 100 | 0 | 0 | 1,2,4 |
| Columbia | 2 | 2 | 100 | 0 | 0 | 6 |
| India | 3 | 3 | 100 | 0 | 0 | 3 |
| Africa | 3 | 3 | 100 | 0 | 0 | 5,13 |
| Total | 21 | 21 | 100 | 0 | 0 | |

B. Sera from Normal Patients and Other Diseases

| SERA | SERUM TESTED | #FALSE POSITIVE | %FALSE POSITIVE | SOURCE[+] |
|------|--------------|-----------------|-----------------|-----------|
| Cutaneous | | | | |
| (old world) | 4 | 0 | 0.0 | 14 |
| (new world) | 13 | 3 | 23.0 | 2,6 |
| Chagas' | 15 | 1 | 6.6 | 1 |
| Malaria | 5 | 0 | 0.0 | 3,8 |
| Leprosy | 4 | 1 | 25.0 | 7 |
| African trypanosomiasis | 4 | 0 | 0.0 | 12 |
| Amoebiasis | 2 | 0 | 0.0 | 11 |
| SLE[*] | 1 | 0 | 0.0 | 10 |
| Schistosomiasis | 3 | 0 | 0.0 | 9 |
| Endemic normal | 14 | 0 | 0.0 | 1,3 |
| Non-endemic normal | 2 | 0 | 0.0 | |
| Other | 4 | 0 | 0.0 | 3,5 |
| Total | 71 | 5 | 7.0 | |

[+] See Table II for key.
[*] Systemic lupus erythematosus

Legends to Figures:

Figure 1. Percent monclonal antibody binding to <u>Leishmania donovani</u> parasite lysates at different protein concentrations. Antibody binding is calculated as a percentage of the maximum $Abs_{405}$ obtained for D2 or D13. A. D2 B. D13.

Figure 2. Elution of dp70 from the D13 affinity resin. Western blots were run on the material eluted from the affinity resin with different buffers. a. Membranes prior to purification, b. 1 M Acetic acid, pH 2.2. c. 0.1 M Glycine, pH 2.5, d. 50 mM Diethylamine, pH 9.5, e. 50 mM Diethylamine, pH 11.5.

Figure 3. Purification of gp70-2 by SE-HPLC. Material eluted from the D2-Sepharose 4B affinity resin was separated by size exclusion-HPLC on a TSK G3000 SW column, 10 µm particle size in 0.1 M PBS containing 0.1% SDS. Fractions were collected every minute and examined for antigenic activity by a dot blot assay. Insert. SDS-PAGE analysis of purification. A. Membranes prior to purification. B. Elution from D2 affinity resin. C. Fractions 11 and 12. D. SDS-PAGE sample buffer only.

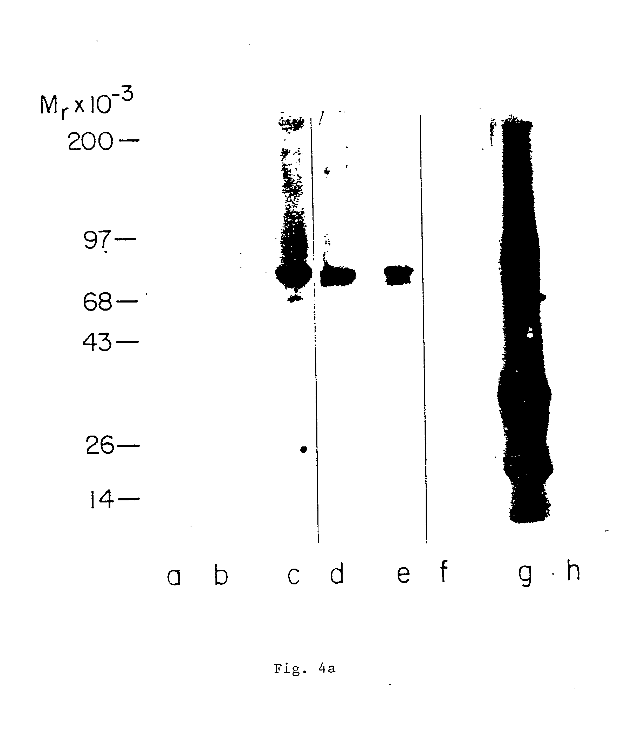

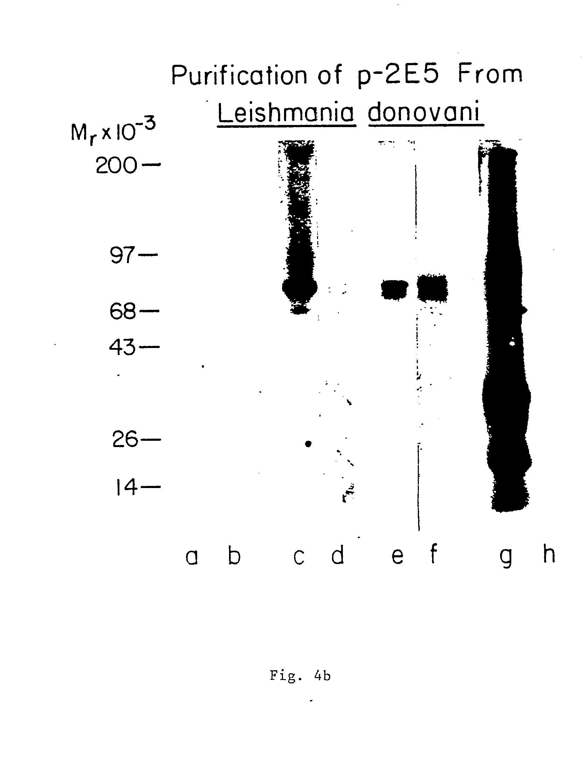

Figure 4. Western blot analysis of purified proteins gp70-2 and dp72 from Leishmania donovani. Crude promastigote membranes and proteins purified by affinity chromatography were separated by SDS-PAGE, transferred to nitrocellulose paper and probed with monoclonal antibodies. Negative control, NS-1 supernatant - lanes a,b; D13 - lanes c,d; D2 - lanes e,f; X4 - lanes g,h. A. Crude membranes L. donovani lanes a,c,e and g; pure dp72 lanes b,d,f, and h. B. Crude membranes L. donovani lanes as in A. Pure gp70-2 lanes b,d,f and h.

Figure 5. Peptide mapping of [35S]-methionine labelled dp72 and gp70-2 with S. aureus V8 protease. A dp72 and B. gp70-2. S. aureus, V8 protease concentration lane a, 0 µg; lane b. 1 µg and lane c. 10 µg.

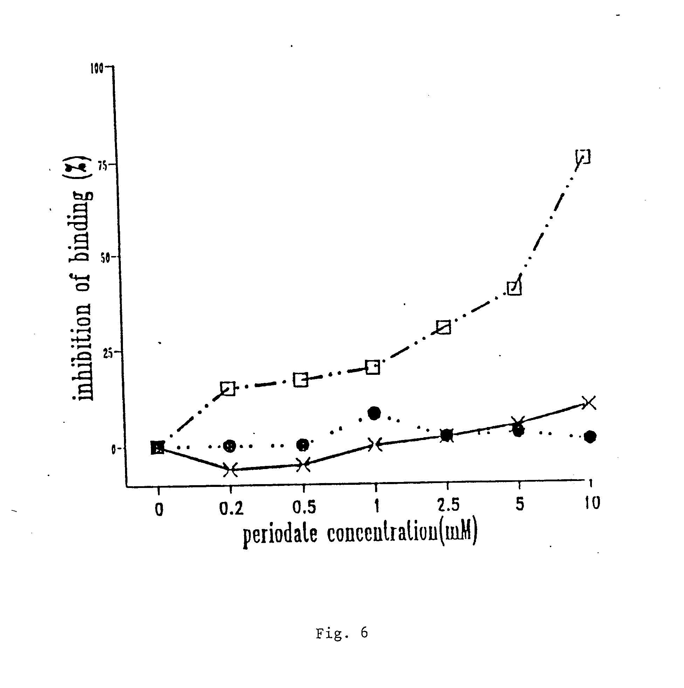

Figure 6. Effect of periodate oxidation on monoclonal antibody to L. donovani antigen. Monoclonal antibody. D2 ⊟ ; D13 X and X4 ●.

Figure 7. Dot blot of human sera reactivity with purified L. donovani proteins. Top Row dp72, Bottom Row gp 70-2. Sera a. visceral leishmaniasis, b. Chagas' disease, c. normal endemic control, d. cutaneous leishmaniasis.

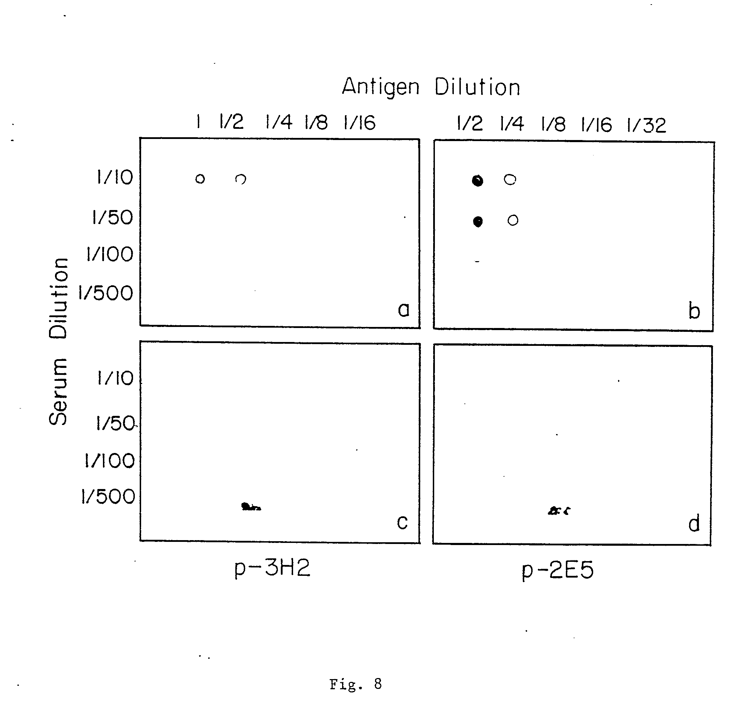

Figure 8. Titration of Human Sera Reaction with Purified Parasite Proteins. a,c. protein dp72; b,d protein gp70-2. Serum from a patient with visceral leishmaniasis in panels a and b. Serum from a patient with Chagas' disease in panals c and d.

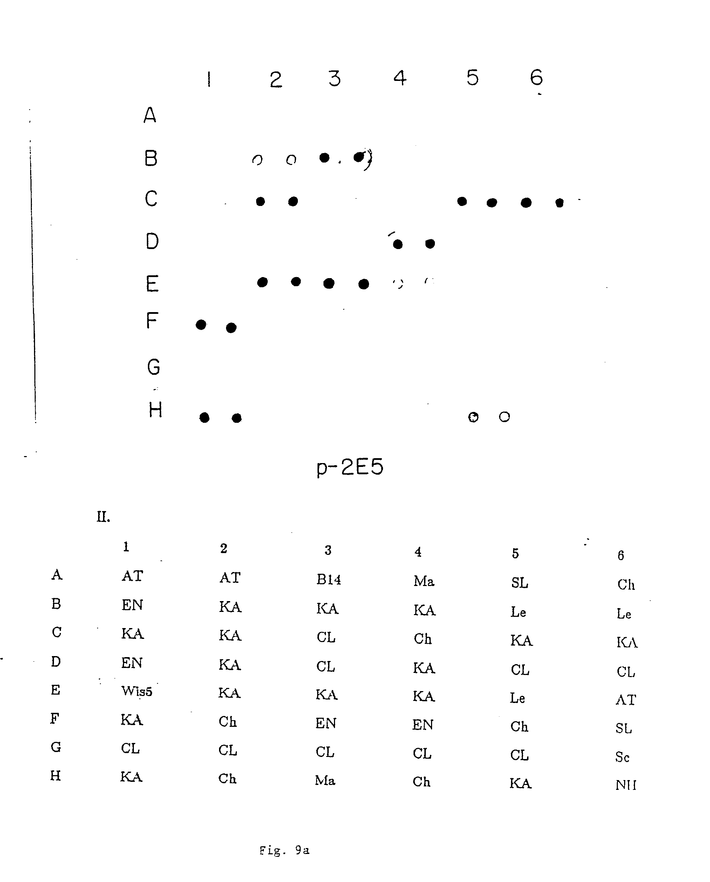

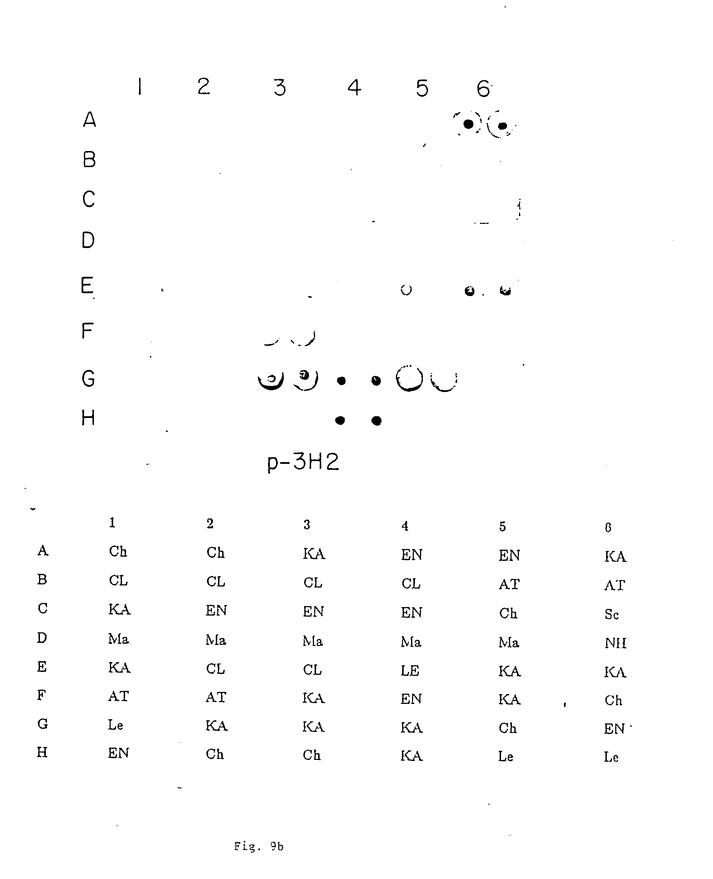

Figure 9. Dot-blot Assay of Human Sera. a. Antigen gp70-2. b. Antigen dp72. Each serum reaction was carried out in duplicate. I. Dot-blot assay. II. Key to serum. AT - African trypanosomiasis, EN - endemic normal human serum, Ma -malaria, SL - systemic lupus erythomatosus, CH - Chagas' disease, CL - cutaneous or mucocutaneous leishmaniasis, Le -leprosy, KA - visceral leishmaniasis, Sc - schistosomiasis and NH - normal human sera from an nonendemic region.

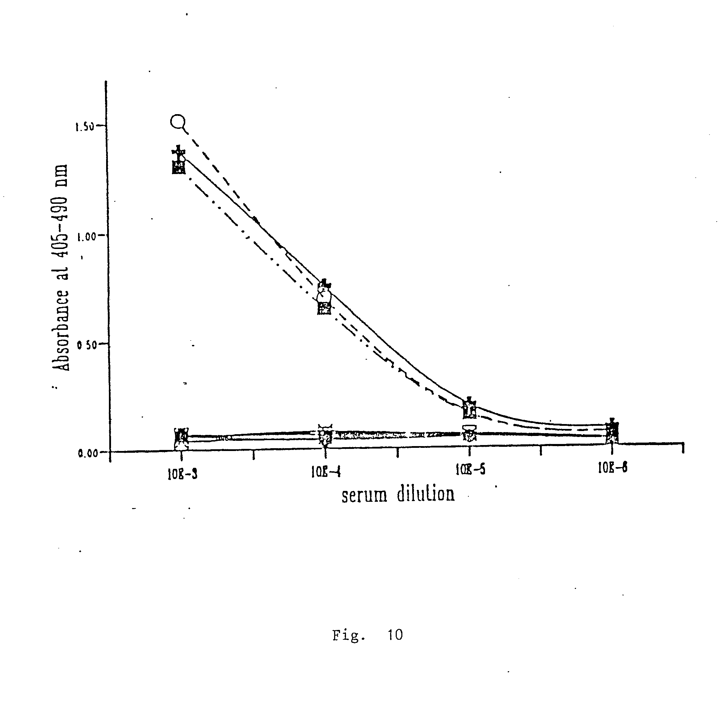

Figure 10. Titration of Human Sera Antibody Binding to Crude Leishmania donovani parasite antigens. O Kala azar sera India; + and kala azar sera - Columbia; O, and X and suspected kala azar sera - India and Africa. Normal human sera - Boston.

## Claims

1. A purified protein derived from Leishmania donovani, having essentially a protein composition as set out in Table I, having a M.W. in the range of from about 66,000 to about 72,000 which reacts with monoclonal antibodies raised against Leishmania donovani, or part of such protein having essentially the antigenic properties of the said protein.

2. A purified protein according to Claim 1, designated as dp72 or such protein designated as gp70-2 which reacts with the monoclonal antibodies designated as D1, D2 or D3, raised against Leishmania donovani.

3. Purified proteins defined in claim 1, substantially as hereinbefore described.

4. The use of an antigen claimed in any of claims 1 to 3 as antigen for the determination of the presence or absence of Leishmaniasis in a body fluid of a human or a canine.

5. An assay for the detection of Leishmaniasis which comprises contacting a body fluid of the person or dog to be examined with an antigen defined in any of claims 1 to 4 and reading the results.

6. An assay according to claim 5, in the form of an antigen dot-ELISA assay.

7. An assay according to claim 5, in the form of a competitive ELISA assay.

8. An assay according to any of claims 5 to 7 where the antigen used is dp72.

9. An assay according to any of claims 5 to 7 where the antigen is purified protein gp70-2.

10 An assay according to any of claims 5 to 9, where the quantity of antigen used is of the order of 50 to 500 ng.

11 Assays for the detection of leishmaniasis and for the quantative determination of the titer of antibodies in the body fluids of the person or dog examined, substantially as hereinbefore described and with references to the Examples.

12 An assay according to any of claims 5 to 9, where the antigen is an antigenically effective part of dp72 or of gp70-2.

13 Assay for the detection of Leishmaniasis, substantially as described.

13

14 The use of a protein claimed in any of claims 1 to 3 as active ingredient of a vaccine against Leishmanias.s

15 The use of a part of the protein designated as dp72 or as gp70-2 as active ingredient of a vaccine against Leishmaniasis

16 Vaccine against Leishmaniasis comprising an effective amount of a protein claimed in any of claims 1 to 3 as active ingredient.

17 Vaccine against Leishmaniasis as claimed in claim 16, where the effective amount of protein is from 0.1 to 5.0 mg per dosage.

18 A protein corresponding essentially to a protein claimed in any of claims 1 to 3, or a protein having essentially identical antigenic and immunogenic properties, whenever produced by genetic engineering.

19 The use of a protein claimed in claim 17 as antigen in an assay for the determination of Leishmaniasis or for use as active ingredient in a vaccine against Leishmaniasis.

Fig. 1

Elution of 70 kD Protein
From Affinity Resin by pH

$M_r \times 10^3$

200—

96—

68—

43—

25—

18—

a   b   c   d   e

Fig. 2

Fig. 3

$M_r \times 10^{-3}$

200 —

97 —

68 —

43 —

26 —

14 —

a  b  c  d  e  f  g · h

Fig. 4a

Purification of p-2E5 From
Leishmania donovani

$M_r \times 10^{-3}$

200 —

97 —

68 —

43 —

26 —

14 —

a   b   c   d   e   f   g   h

Fig. 4b

Fig. 5

Fig. 6

Dot-Blot Serodiagnosis of Human Kala-azar
on Purified <u>Leishmania donovani</u>
Antigen

Fig. 7

Fig. 8

p-2E5

II.

| | 1 | 2 | 3 | 4 | 5 | 6 |
|---|---|---|---|---|---|---|
| A | AT | AT | B14 | Ma | SL | Ch |
| B | EN | KA | KA | KA | Le | Le |
| C | KA | KA | CL | Ch | KA | KA |
| D | EN | KA | CL | KA | CL | CL |
| E | Wis5 | KA | KA | KA | Le | AT |
| F | KA | Ch | EN | EN | Ch | SL |
| G | CL | CL | CL | CL | CL | Sc |
| H | KA | Ch | Ma | Ch | KA | NH |

Fig. 9a

p-3H2

|     | 1   | 2   | 3   | 4   | 5   | 6   |
|-----|-----|-----|-----|-----|-----|-----|
| A   | Ch  | Ch  | KA  | EN  | EN  | KA  |
| B   | CL  | CL  | CL  | CL  | AT  | AT  |
| C   | KA  | EN  | EN  | EN  | Ch  | Sc  |
| D   | Ma  | Ma  | Ma  | Ma  | Ma  | NH  |
| E   | KA  | CL  | CL  | LE  | KA  | KA  |
| F   | AT  | AT  | KA  | EN  | KA  | Ch  |
| G   | Le  | KA  | KA  | KA  | Ch  | EN  |
| H   | EN  | Ch  | Ch  | KA  | Le  | Le  |

Fig. 9b

Fig. 10